# EUROPÄISCHE PATENTSCHRIFT

(11) **EP 0 486 885 B1**
(45) Veröffentlichungstag und Bekanntmachung des Hinweises auf die Patenterteilung: **29.05.1996**
(21) Anmeldenummer: 91118965.2
(22) Anmeldetag: 07.11.1991
(51) Int. Cl.: C07C 59/64

(54) **Verwendung von Arylpolyencarbonsäuren und ihren Derivaten als Lichtschutzmittel in kosmetischen Zubereitungen**
Utilization of arylpolyencarboxylic acids and their derivatives as light protection agents in cosmetic compositions
Utilisation d'acides arylpolyencarboxyliques et leur dérivés comme agents de protection contre la lumière dans les compositions cosmétiques

(30) Priorität: 17.11.1990 DE 4036779
(43) Veröffentlichungstag der Anmeldung: 27.05.1992
(73) Patentinhaber: BASF Aktiengesellschaft, D-67063 Ludwigshafen (DE)
(72) Erfinder: Deckers, Gabriele, Dr., W-6700 Ludwigshafen (DE); Becker, Rainer, Dr., W-6702 Bad Duerkheim (DE); Wenderoth, Bernd, Dr., W-6840 Lampertheim (DE); Goetz, Norbert, Dr., W-6520 Worms 1 (DE); Sperling-Vietmeier, Karin, Dr., W-6730 Neustadt (DE)

(56) Entgegenhaltungen:
- EP-A- 0 169 571
- Zeitschr. f. Anylyt. Chemie, 207, (1965), S. 270 ff.
- Archiv d. Pharmazie, 300 (1967), S. 157 ff.
- Chem. Berichte, 62, (1929), S. 360 ff.
- J. Org. Chem., 41 (1976), S. 4070 ff.
- DE-OS 36 15 157
- DE-OS 37 37 399

## Beschreibung

Die vorliegende Erfindung betrifft die Verwendung von Arylpolyencarbonsäuren und ihren Derivaten der allgemeinen Formel I 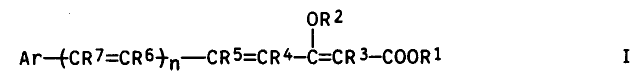
in der die Variablen folgende Bedeutung haben:
- Ar: ein Phenyl-, Biphenylyl- oder Naphthylrest, der durch ein bis drei C₁- bis C₄-Alkylgruppen, C₁- bis C₄-Alkoxygruppen, Hydroxylgruppen, Phenoxygruppen, Aminogruppen, welche durch C₁- bis C₄-Alkylgruppen mono- oder disubstituiert sein können, oder eine Methylendioxygruppe substituiert sein kann, wobei die Substituenten gleich oder verschieden sein können,
- R¹: Wasserstoff, ein Alkalimetall- oder Ammoniumkation, eine C₁- bis C₂₀-Alkylgruppe oder eine C₂- bis C₂₀-Alkenylgruppe,
- R²: eine C₁- bis C₈-Alkylgruppe,
- R³ bis R⁷: Wasserstoff oder C₁- bis C₄-Alkylgruppen und
- n: 0 oder 1
als Lichtschutzmittel in kosmetischen Zubereitungen.

Ein Teil der Arylpolyencarbonsäuren und Derivate I sind neue Verbindungen.

Weiterhin betrifft die Erfindung die Verbindungen I enthaltende kosmetische Zubereitungen.

Die in kosmetischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, spezielle durch UV-A-Strahlung, hervorgerufen werden. All diese Erkenntnisse lassen daher die Entwicklung effizienter Filtersubstanzen für den UV-A-Bereich notwendig erscheinen.

Es besteht ein wachsender Bedarf an Lichtschutzmitteln für kosmetische Zubereitungen, die vor allem als UV-A-Filter dienen können und deren Absorptionsmaxima deshalb im Bereich von ca. 320 bis 380 nm liegen sollten. Um mit einer möglichst geringen Einsatzmenge die gewünschte Wirkung zu erzielen, sollten derartige Lichtschutzmittel zusätzlich eine hohe spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen, hohe Stabilität der mit ihnen hergestellten Emulsionen, hohe Photostabilität sowie geringen Eigengeruch und geringe Eigenfärbung.

In Archiv der Pharmazie, 300 (1967), Seite 157 - 169, werden UV-absorptiometrische Untersuchungen von Inhaltsstoffen der Kawadroge, beispielsweise Kawasäure, Methysticinsäure und Methysticinsäuremethylester beschrieben.

So werden in der DE-A 32 06 586 (1) 2-Acetyl-5-phenyl-2,4-pentadiensäure-ester der Formel II 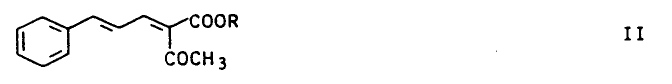
beschrieben. Diese Verbindungen werden als Lichtschutzmittel für kosmetische und technische Anwendungen empfohlen.

Die DE-A 27 28 242 (2) betrifft kosmetische Lichtschutzmittel für den UV-A-Bereich mit einem Gehalt an Brenztraubensäure- bzw. Lävulinsäure-Kondensationsprodukten der Formel III 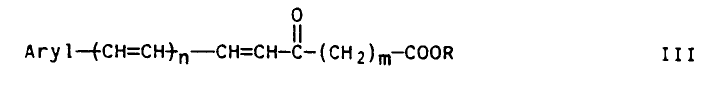
in der n 0 oder 1 und m 0 oder 1 oder 2 bezeichnet.

Die DE-A 29 45 125 (3) betrifft 4-tert.-Butyl-4'-methoxy-dibenzoylmethan (IV) und Lichtschutzmittel zur Verzögerung der Hautalterung, welche IV enthalten.

Die Verbindungen II bis IV genügen den oben genannten Anforderungen an UV-Filtersubstanzen für kosmetische Anwendungen jedoch nur bedingt.

Der vorliegenden Erfindung lag daher die Aufgabe zugrunde, Lichtschutzmittel für kosmetische Zubereitungen bereitzustellen, die den genannten Anforderungen in höherem Maße gerecht werden als die bekannten Mittel dieser Art.

Demgemäß wurde die eingangs definierte Verwendung von Arylpolyencarbonsäuren und ihren Derivaten I als Lichtschutzmittel in kosmetischen Zubereitungen gefunden.

Für den Arylrest kommt weiterhin insbesondere ein Phenylrest, der durch ein bis drei C₁- bis C₄-Alkylgruppen, z.B. Methyl, Ethyl, n-Propyl, iso-Propyl, n-Butyl oder tert.-Butyl, oder C₁- bis C₄-Alkoxygruppen, z.B. Methoxy oder Ethoxy, oder eine Methylendioxygruppe oder eine Di(C₁- bis C₄-alkyl)aminogruppe, z.B. Dimethylamino oder Diethylamino, substituiert sein kann, in Betracht.

Als Beispiele für Ar sind zu nennen:
Phenyl,
o- oder p-Tolyl,
p-tert.-Butyl,
2,3-, 2,4-, 2,5-, 2,6-, 3,4- oder 3,5-Dimethylphenyl,
Mesityl,
o- oder p-Methoxyphenyl,
2,3-, 2,4-, 2,5-, 2,6-, 3,4- oder 3,5-Dimethoxyphenyl,
3,4,5-Trimethoxyphenyl und
3,4-Methylendioxyphenyl.

Der Rest R¹ steht für Wasserstoff oder ein Alkalimetallkation, z.B. Natrium oder Kalium, oder das Ammoniumkation oder er steht für eine geradkettige oder verzweigte C₁- bis C₂₀-Alkylgruppe oder eine C₂- bis C₂₀-Alkenylgruppe. Im ersten Fall handelt es sich um Carbonsäuren bzw. deren Salze, im letzteren Fall um Carbonsäureester.

In einer bevorzugten Ausführungsform steht R¹ für Wasserstoff oder eine geradkettige oder verzweigte C₁- bis C₈-Alkylgruppe wie Methyl, Ethyl, n-Propyl, iso-Propyl, n-Butyl, iso-Butyl, sec.-Butyl, tert.-Butyl, n-Pentyl, iso-Pentyl, n-Hexyl, n-Heptyl, n-Octyl oder 2-Ethylhexyl.

Der Rest R², welcher in Form eines β-Alkoxyrestes ein essentielles Strukturmerkmal in den Verbindungen I darstellt, bezeichnet eine C₁- bis C₈-Alkylgruppe, vorzugsweise eine C₁- bis C₄-Alkylgruppe wie Methyl, Ethyl, n-Propyl, iso-Propyl, n-Butyl, iso-Butyl, sec.-Butyl, oder tert.-Butyl.

Die Reste R³ bis R⁷ bedeuten unabhängig voneinander Wasserstoff oder C₁- bis C₄-Alkylgruppen, insbesondere Wasserstoff oder Methylgruppen.

Die Verbindungen I können als cis-trans-Isomerengemische bezüglich der olefinischen Doppelbindungen oder als isomerenreine Verbindungen vorliegen.

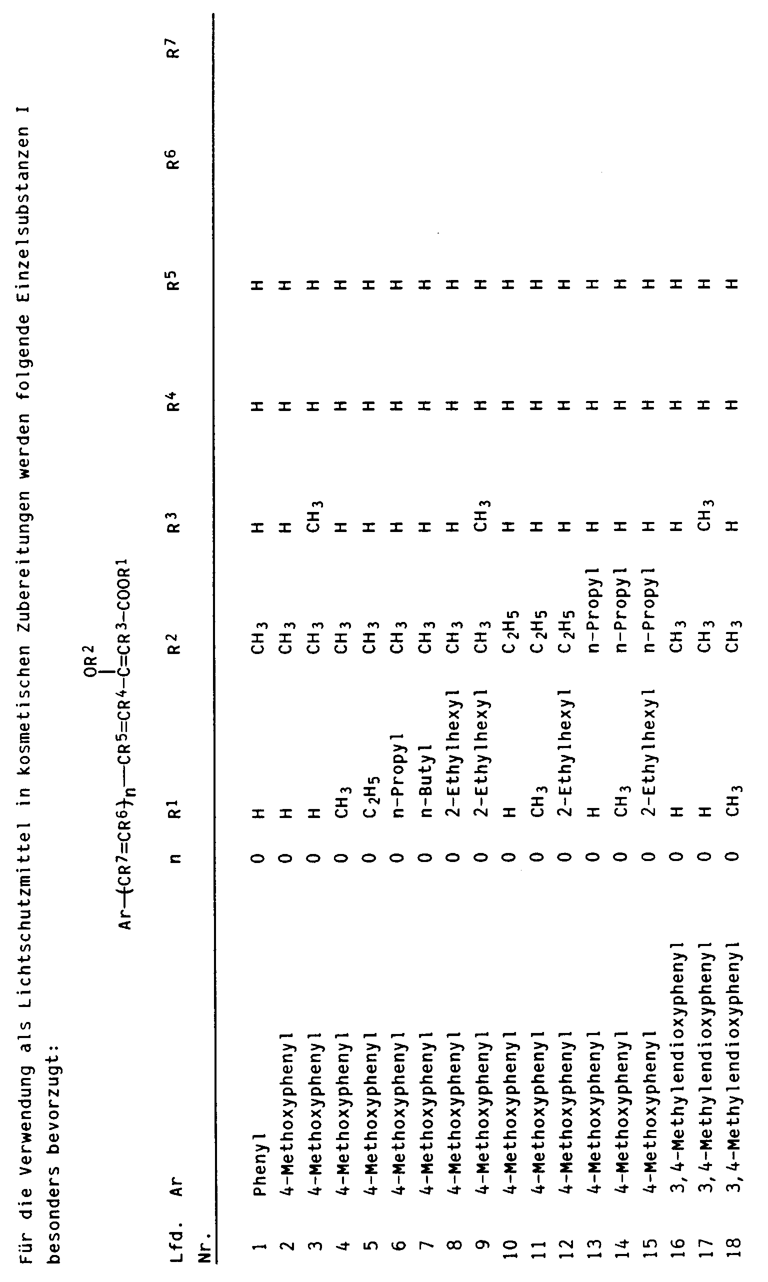

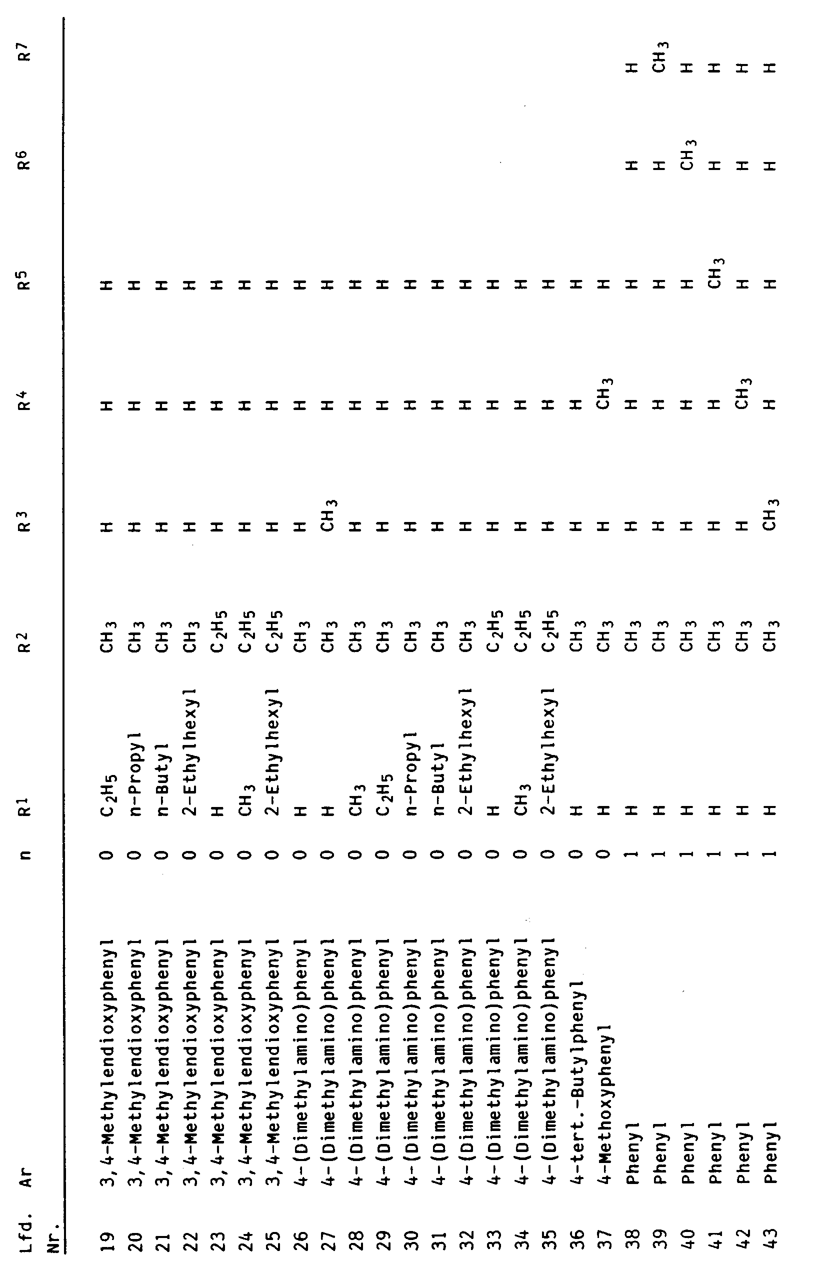

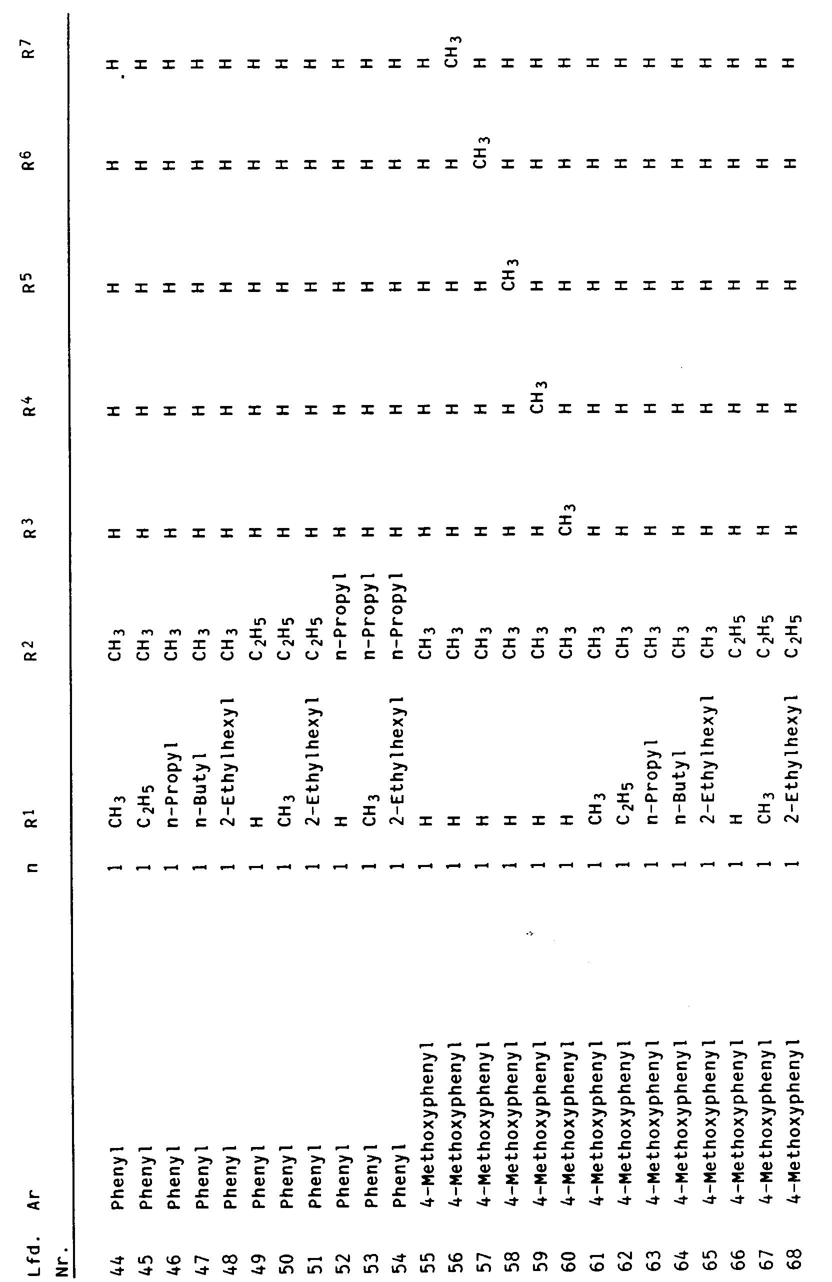

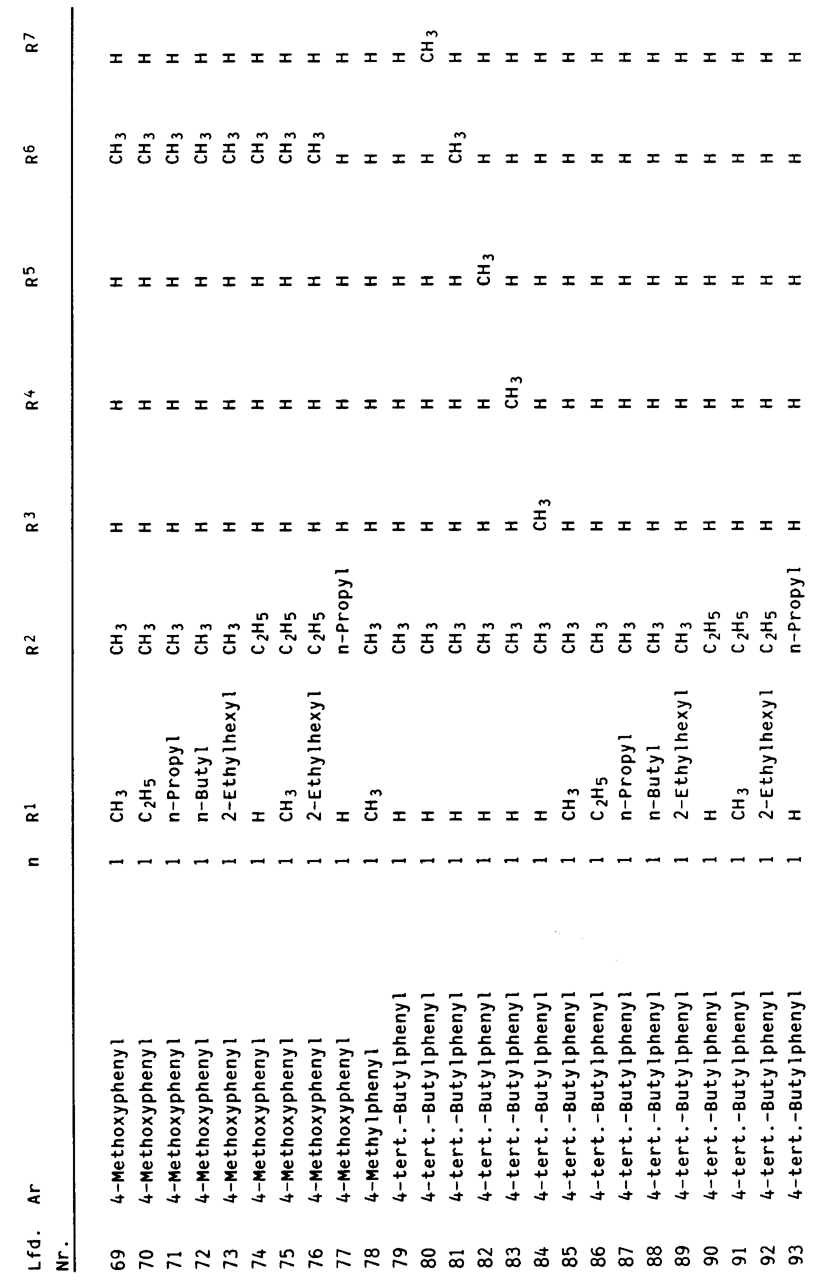

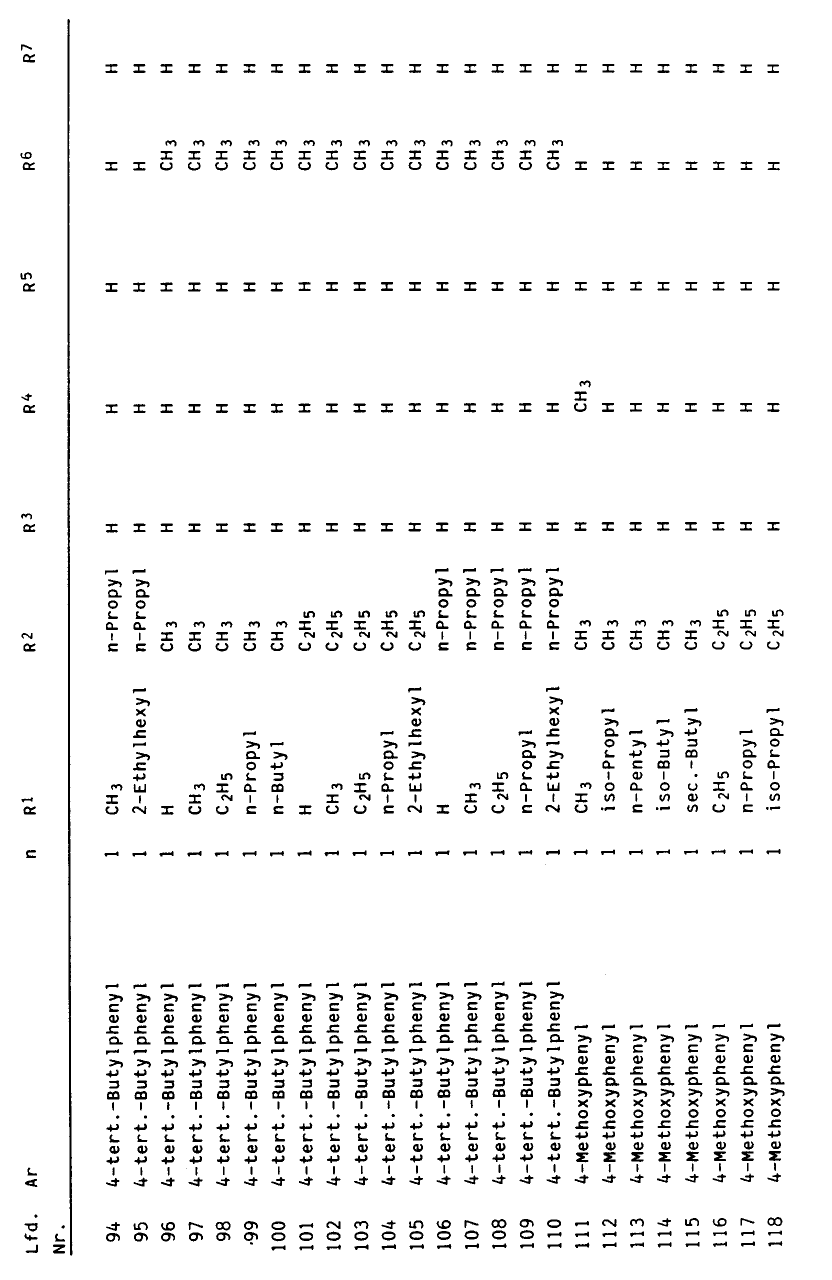

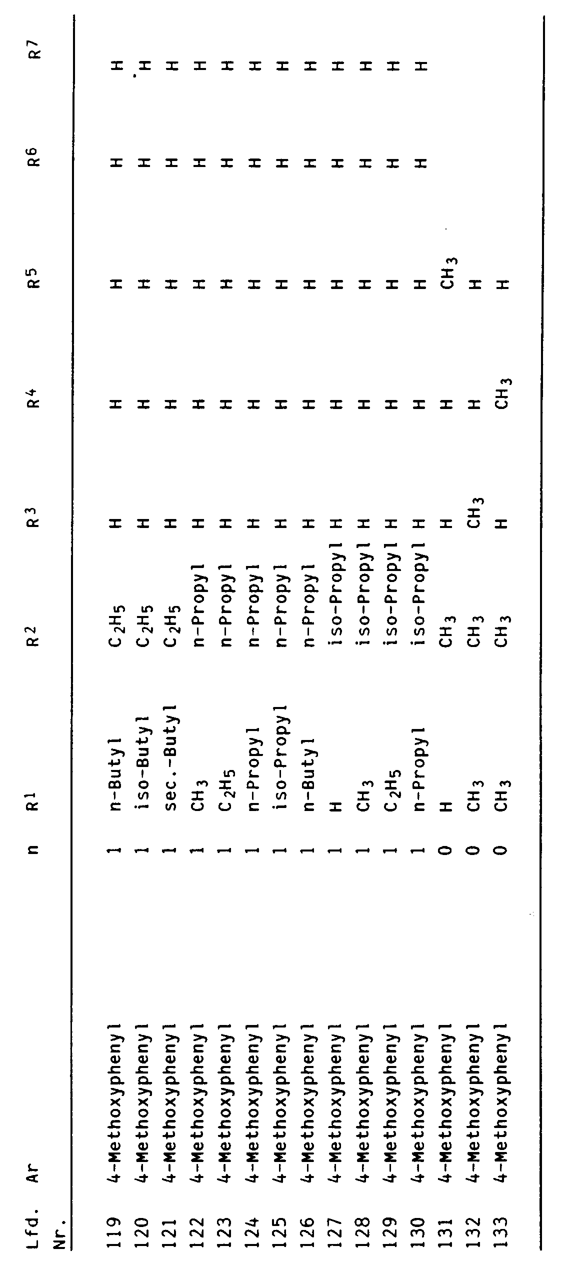

Ein Teil der Arylpolyencarbonsäuren und ihrer Derivate I sind neue Verbindungen. Deshalb betrifft die vorliegende Erfindung weiterhin Arylpolyencarbonsäuren und ihre Derivate der allgemeinen Formel Ia 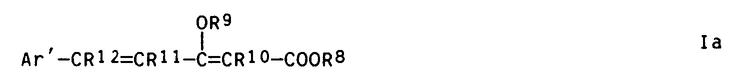
wobei die Variablen Ar und Ar', R¹ und R⁸, R² und R⁹ sowie R³ bis R⁵ und R¹⁰ bis R¹² die gleiche Bedeutung haben, mit Ausnahme folgender Einzelverbindungen für die Arylpolyencarbonsäuren und Derivate Ia: 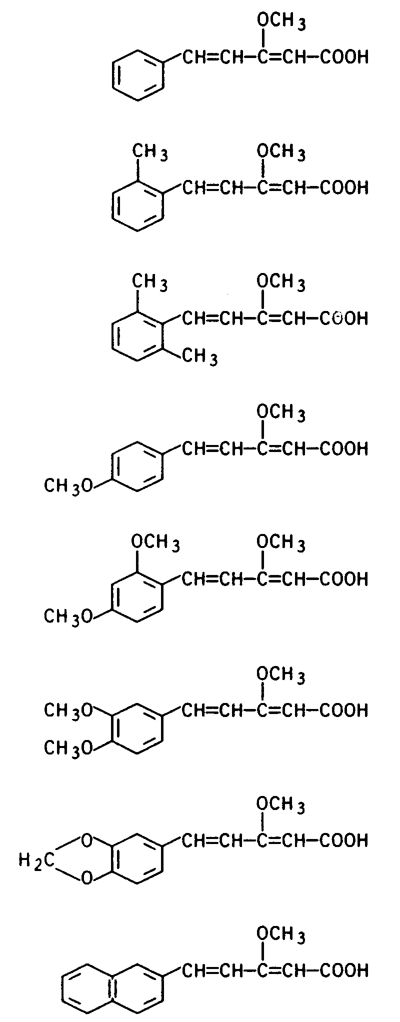 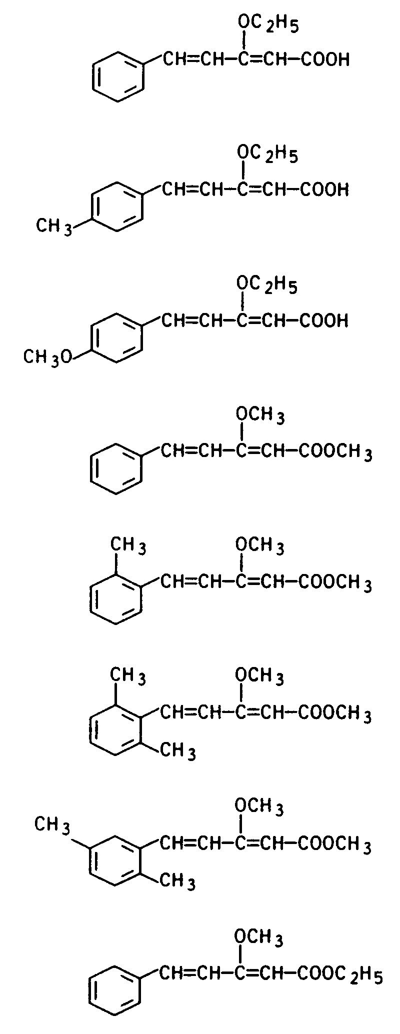
Weiterhin betrifft die vorliegende Erfindung Arylpolyencarbonsäuren und ihre Derivate der allgemeinen Formel Ib 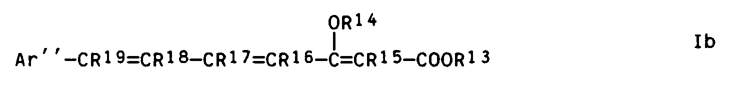
wobei die Variablen Ar und Ar'', R¹ und R¹³, R² und R¹⁴ sowie R³ bis R⁷ und R¹⁵ bis R¹⁹ die gleiche Bedeutung haben, mit Ausnahme folgender Einzelverbindungen für die Arylpolyencarbonsäuren und Derivate Ib: 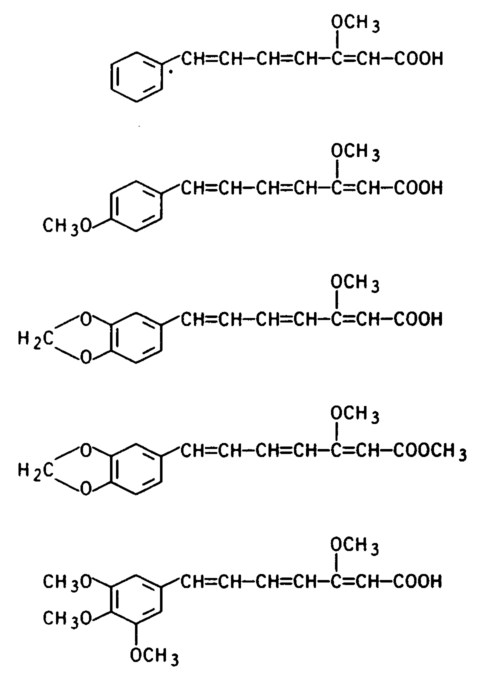
Von besonderem Interesse sind diejenigen neuen Arylpolyencarbonsäuren und Derivate Ia und Ib, welche in der Liste der Einzelsubstanzen Nr. 1 bis 133 aufgeführt sind, insbesondere jedoch diejenigen, welche hiervon als experimentelle Beispiele mit Schmelzpunkt und spektroskopischen Daten genannt sind.

Die erfindungsgemäßen Verbindungen I werden zweckmäßigerweise durch Aldol-Kondensation eines Aldehyds oder Ketons der allgemeinen Formel IV 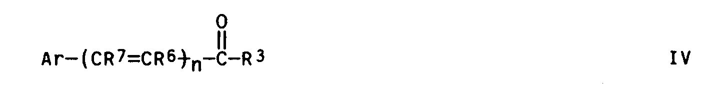
mit einem β-Alkoxycarbonsäure-Derivat der allgemeinen Formel V 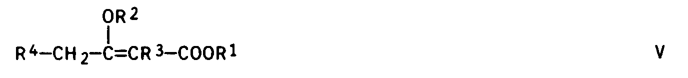
in an sich bekannter Weise unter basischen Reaktionsbedingungen hergestellt.

Je nach Reaktionsführung werden hierbei in der Regel zunächst die freien Säuren oder deren Salze (R¹ = Wasserstoff bzw. ein Alkalimetall- oder Ammoniumkation) gebildet, die dann durch übliche Umsetzungen mit den entsprechenden Alkoholen zu Estern (R¹ = C₁- bis C₂₀-Alkyl oder C₂- bis C₂₀-Alkenyl) derivatisiert werden können.

Die Aldol-Kondensation wird normalerweise in einem inerten organischen Lösungsmittel wie einem Kohlenwasserstoff, z.B. Toluol oder Xylol, oder einem Ether, z.B. Diethylether, Methyl-tert.-butylether, Diisopropylether, Tetrahydrofuran oder Dioxan, durchgeführt. Als Basen kommen beispielsweise Natriumhydrid, Natriumamid, Lithiumdiisopropylamid, Natriummethylat, Natriumethylat oder Kalium-tert.-butylat in Betracht. Die Reaktionstemperaturen liegen üblicherweise zwischen 0 und 150°C, vorzugsweise zwischen 20 und 100°C, insbesondere zwischen 40 und 80°C. In der Regel arbeitet man bei Normaldruck.

Die erfindungsgemäß zu verwendenden Verbindungen I dienen als Lichtschutzmittel in kosmetischen Zurbereitungen zur vorsorglichen Sonnenschutzpflege. Solche Sonnenschutzpräparate können in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Gegenstand der vorliegenden Erfindung sind weiterhin kosmetische Zubereitungen, die 0,1 bis 10 Gew.-%, vorzugsweise 1 bis 7 Gew.-%, bezogen auf die gesamte Menge der kosmetischen Zubereitung, einer oder mehrerer der Verbindungen I als Lichtschutzmittel enthalten, wobei die Verbindungen I in üblichen Trägerstoffen oder Verdünnungsmitteln eingesetzt werden, beispielsweise als Lösung in einem Öl.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Zusammen mit den Verbindungen I können weitere übliche Lichtschutzmittel wie
2-Hydroxy-4-methoxybenzophenon,
2-Hydroxy-4-methoxybenzophenon-5-sulfonsäure,
mit 25 mol Ethylenoxid umgesetzter p-Aminobenzoesäureethylester,
p-Methoxyzimtsäure-2-ethylhexylester,
p-(N,N-Dimethylamino)benzoesäure-2-ethylhexylester,
2-Phenylbenzimidazol-5-sulfonsäure,
3-(4-Methylbenzyliden)campher oder
2,4,6-Trianilino-p-(carbo-2'-ethylhexyl-1'-oxy)-1,3,5-triazin
in den hierfür üblichen Mengen mitverwendet werden.

Übliche kosmetische Hilfsstoffe, die als Zusätze in Betracht kommen können, sind z.B. Emulgatoren wie Fettalkoholethoxylate, Sorbitanfettsäureester oder Lanolinderivate, Verdickungsmittel wie Carboxymethylcellulose oder vernetzte Polyacrylsäure, Konservierungsmittel und Parfüms.

Die erfindungsgemäß zu verwendenden Arylpolyencarbonsäuren und ihre Derivate I zeichnen sich in der Regel durch ein besonders hohes Absorptionsvermögen, insbesondere im Bereich der UV-A-Strahlung, aus, ersichtlich am Vergleich der Extinktionen der erfindungsgemäßen Verbindungen I mit beispielsweise den Extinktionen typischer Vertreter der Verbindungen II bis IV. Sie können auch vorteilhaft in Kombination mit UV-B-Filtersubstanzen verwendet werden. Weiterhin sind sie gut in kosmetischen Ölen löslich und lassen sich leicht in kosmetische Formulierungen einarbeiten. Die mit den Verbindungen I hergestellten Emulsionen zeichnen sich besonders durch ihre hohe Stabilität, die Verbindungen I selber durch ihre hohe Photostabilität, ersichtlich am Vergleich der Photostabilitäten der erfindungsgemäßen Verbindungen I mit beispielsweise den Photostabilitäten typischer Vertreter der Verbindungen II bis IV, und die mit I hergestellten Zubereitungen durch ihr angenehmes Hautgefühl aus.

Die Verbindungen I sind nahezu farblos und geruchlos. Derzeitig verfügbare, handelsübliche UV-A-Filtersubstanzen sind gelb und neigen dazu, auch auf Kleidungsstücke abzufärben. Dies kann durch Verwendung der erfindungsmäßigen Verbindungen vermieden werden.

### Herstellungsbeispiele

### 3-Methoxy-7-(4'-methoxyphenyl)-hepta-2,4,6-triensäure (Nr. 55 der Liste der besonders bevorzugten Einzelverbindungen)

16,5 g einer 80 gew.-%igen Suspension von Natriumhydrid in Weißöl (entsprechend 0,55 mol NaH) wurden mit 500 ml wasserfreiem Tetrahydrofuran vermischt und bei 50 bis 60°C unter gutem Rühren mit einer Mischung aus 81 g (entsprechend 0,5 mol) p-Methoxyzimtaldehyd und 65 g (entsprechend 0,5 mol) β-Methoxycrotonsäuremethylester tropfenweise versetzt. Dabei wurde die Zutropfgeschwindigkeit so gewählt, daß das Gemisch am schwachen Sieden blieb. Anschließend wurde 3 Stunden bei 65°C und dann über Nacht bei Raumtemperatur nachgerührt. Zur Aufarbeitung wurde das Reaktionsgemisch unter Kühlung mit 0,5 l Eiswasser versetzt und danach mit Methyl-tert.-butylether extrahiert. Die verbliebene wäßrig-alkalische Phase wurde mit verdünnter Salzsäure unter Eiskühlung angesäuert, der ausgefallene Niederschlag wurde abfiltriert und gut mit Wasser nachgewaschen. Man erhielt nach Trocknung 83 g der Titelverbindung vom Schmelzpunkt 187°C (nach Umkristallisation aus Essigester), entsprechend einer Ausbeute von 64 %.

Die folgenden Verbindungen der Liste der besonders bevorzugten Einzelverbindungen wurden analog oben angegebener Vorschrift hergestellt. Weiterhin sind in der folgenden Tabelle die längstwelligen Maxima λₘₐₓ sowie die Extinktionen E (1 gew.-%ige Lösung, Schichtdicke 1 cm) der Verbindungen angeführt. Als Vergleich dienen die entsprechenden Werte der Verbindung A gemäß Literaturstelle (1), der Verbindung B gemäß Literaturstelle (2) und der Verbindung C gemäß Literaturstelle (3).

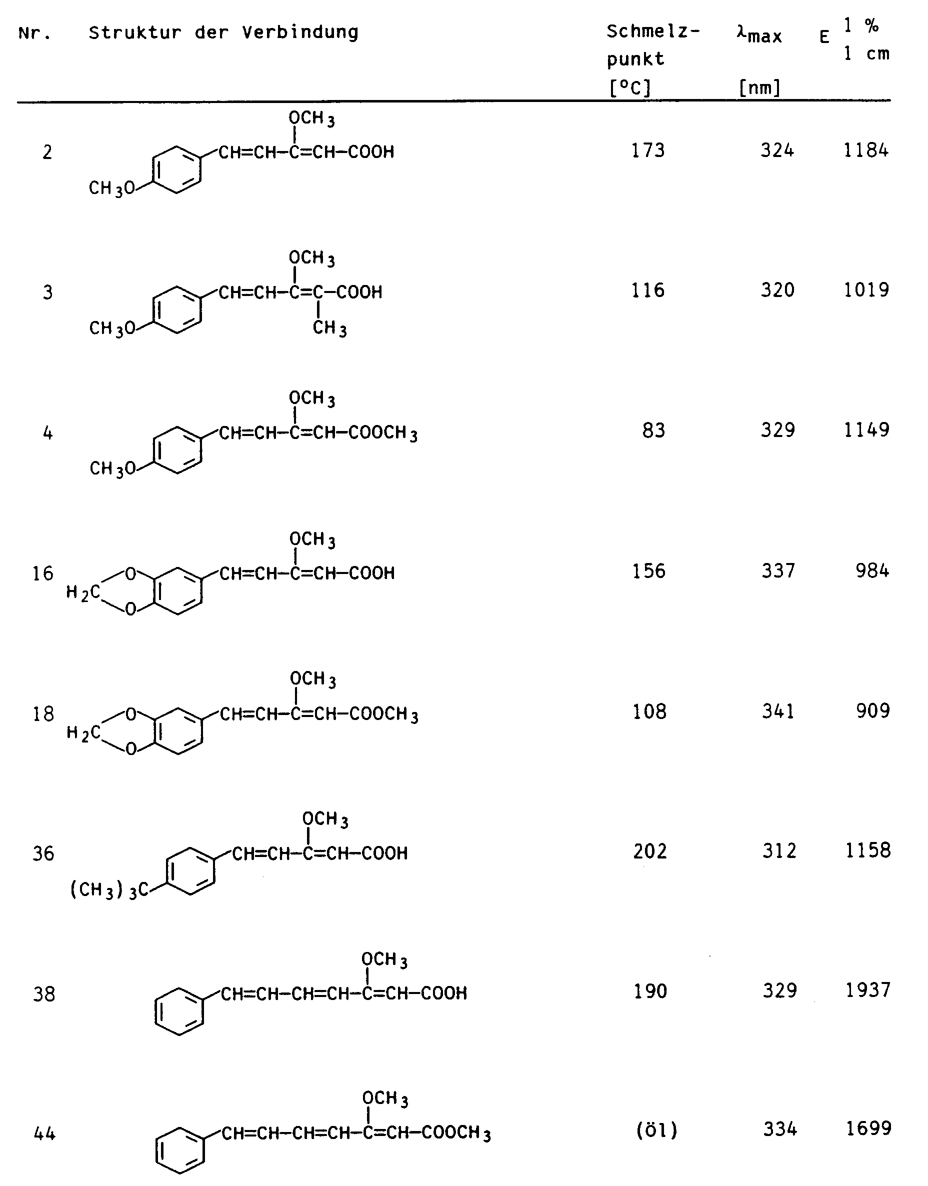

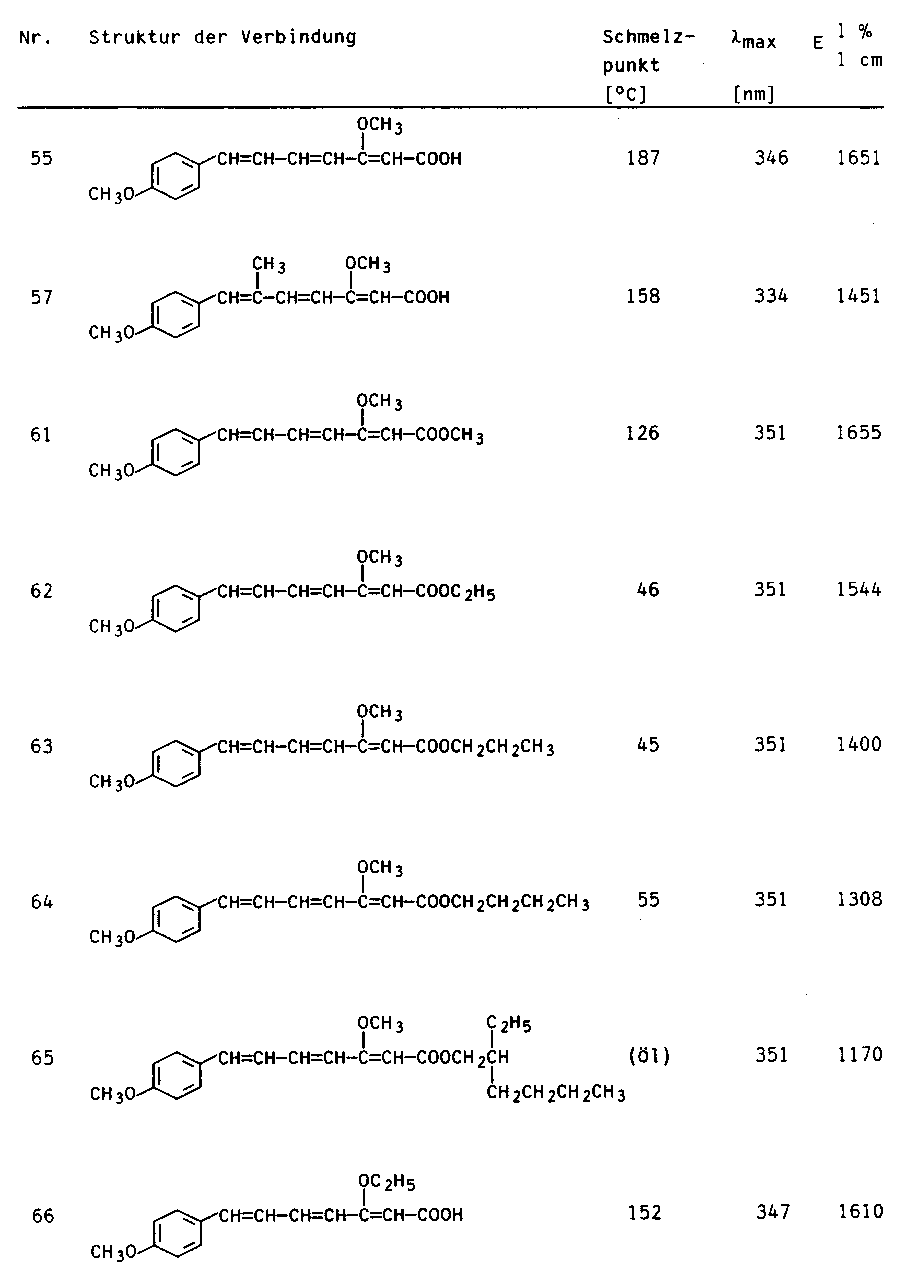

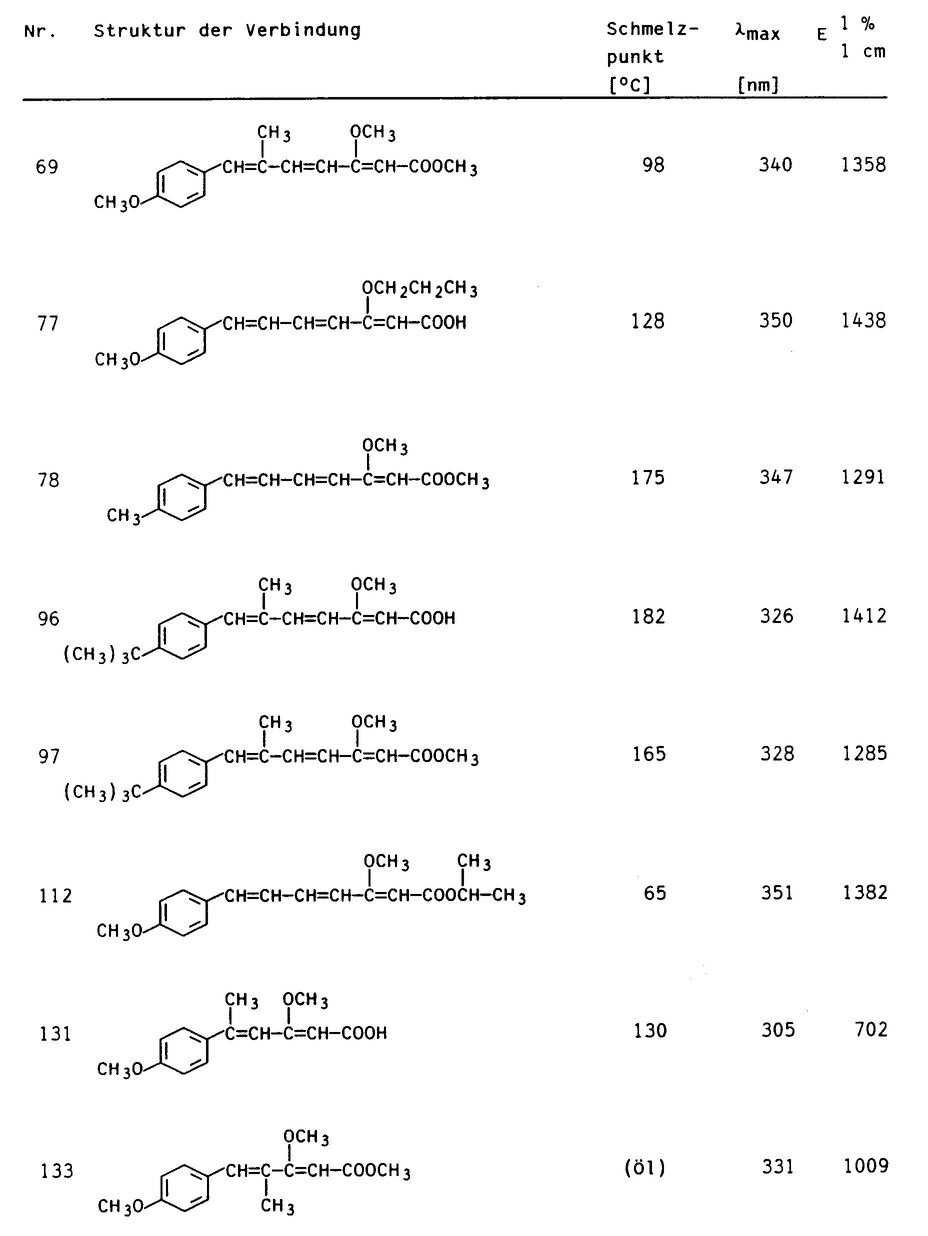

Zum Vergleich:

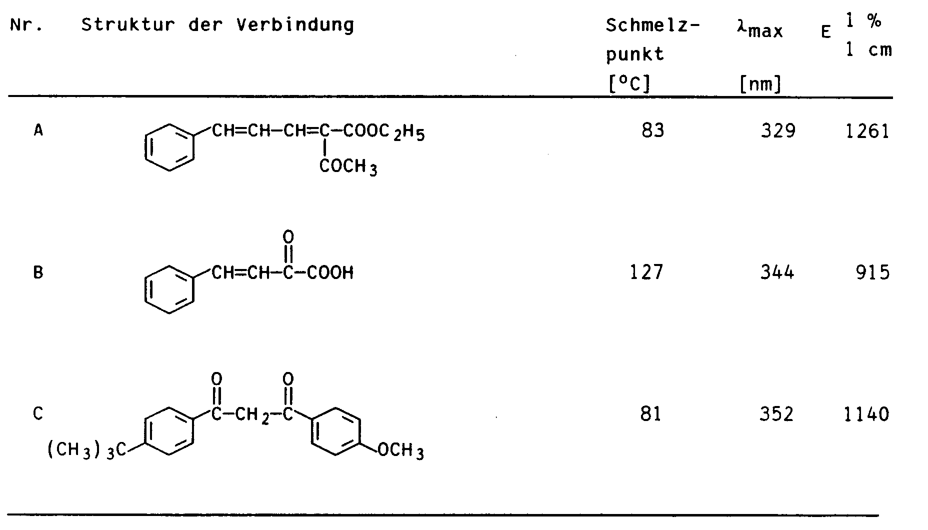

Die erfindungsgemäß zu verwendenden Verbindungen zeigen eine höhere Photostabilität als die bekannten Mittel des Standes der Technik. So weist die Verbindung Nr. 38 nach 2 Stunden Bestrahlung eine Restaktivität von 90 % und nach 8 Stunden Bestrahlung eine Restaktivität von 80 % auf, wogegen die Verbindungen A und B schon nach 2 Stunden Bestrahlung eine Restaktivität von nur noch 30 % bzw. von sogar weniger als 10 % zeigen und die Verbindung C nach 6 Stunden Bestrahlung eine Restaktivität von 51 % hat. Zur Bestimmung der Restaktivität wurde eine ethanolische Lösung der Verbindung mit einem Gehalt von 10 mg/l in einem Suntest® der Firma Heraeus, der eine Xenonlampe als Strahlenquelle enthält, bestrahlt und nach den angegebenen Zeiten auf ihren Gehalt untersucht.

## Patentansprüche (Patentansprüche für folgende(n) Vertragsstaat(en): BE, CH, DE, FR, GB, IT, LI, NL)

1. Verwendung von Arylpolyencarbonsäuren und ihren Derivaten der allgemeinen Formel I 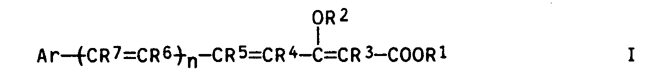 in der die Variablen folgende Bedeutung haben:
Ar ein Phenyl-, Biphenylyl- oder Naphthylrest, der durch ein bis drei C₁- bis C₄-Alkylgruppen, C₁- bis C₄-Alkoxygruppen, Hydroxylgruppen, Phenoxygruppen, Aminogruppen, welche durch C₁- bis C₄-Alkylgruppen mono- oder disubstituiert sein können, oder eine Methylendioxygruppe substituiert sein kann, wobei die Substituenten gleich oder verschieden sein können,
R¹ Wasserstoff, ein Alkalimetall- oder Ammoniumkation, eine C₁- bis C₂₀-Alkylgruppe oder eine C₂- bis C₂₀-Alkenylgruppe,
R² eine C₁- bis C₈-Alkylgruppe,
R³ bis R⁷ Wasserstoff oder C₁- bis C₄-Alkylgruppen und
n 0 oder 1
als Lichtschutzmittel in kosmetischen Zubereitungen.

2. Verwendung von Arylpolyencarbonsäuren und ihren Derivaten I nach Anspruch 1 als Lichtschutzmittel in kosmetischen Zubereitungen, wobei die Variablen folgende Bedeutung haben:
Ar ein Phenylrest, der durch ein bis drei C₁- bis C₄-Alkylgruppen oder C₁- bis C₄-Alkoxygruppen oder eine Methylendioxygruppe oder eine Di(C₁- bis C₄-alkyl)aminogruppe substituiert sein kann,
R¹ Wasserstoff oder eine C₁- bis C₈-Alkylgruppe,
R² eine C₁- bis C₄-Alkylgruppe,
R³ bis R⁷ Wasserstoff oder eine Methylgruppe und
n 0 oder 1.

3. Arylpolyencarbonsäuren und ihre Derivate der allgemeinen Formel Ia 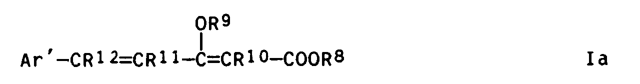 wobei die Variablen Ar und Ar', R¹ und R⁸, R² und R⁹ sowie R³ bis R⁵ und R¹⁰ bis R¹² die gleiche Bedeutung haben, mit Ausnahme folgender Einzelverbindungen für die Arylpolyencarbonsäuren und Derivate Ia:

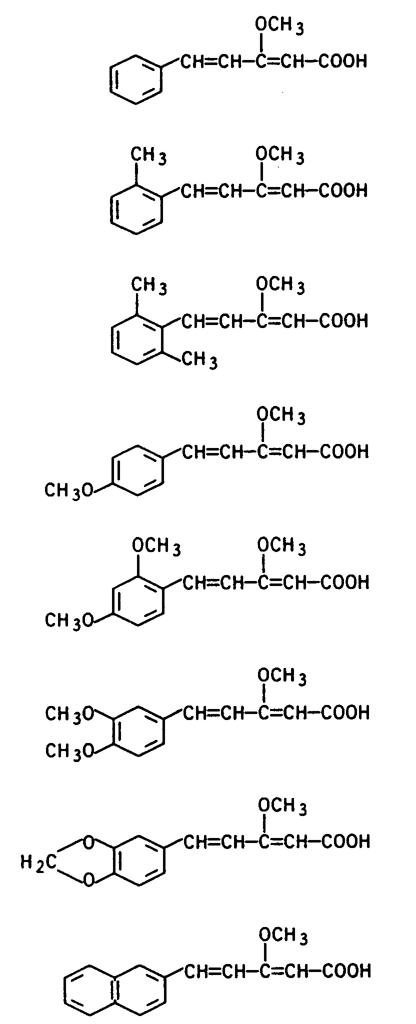

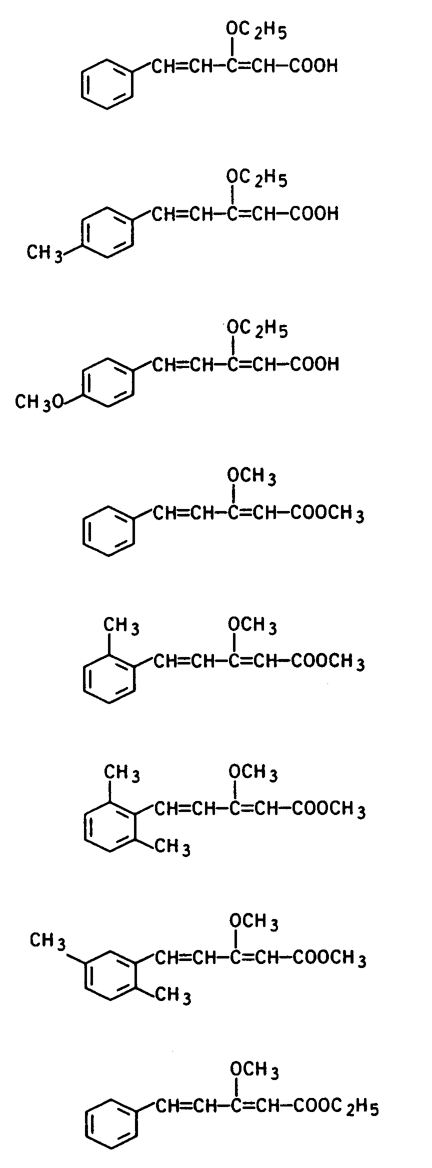

4. Arylpolyencarbonsäuren und ihre Derivate der allgemeinen Formel Ib 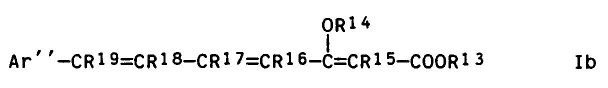 wobei die Variablen Ar und Ar'', R¹ und R¹³, R² und R¹⁴ sowie R³ bis R⁷ und R¹⁵ bis R¹⁹ die gleiche Bedeutung haben, mit Ausnahme folgender Einzelverbindungen für die Arylpolyencarbonsäuren und Derivate Ib:

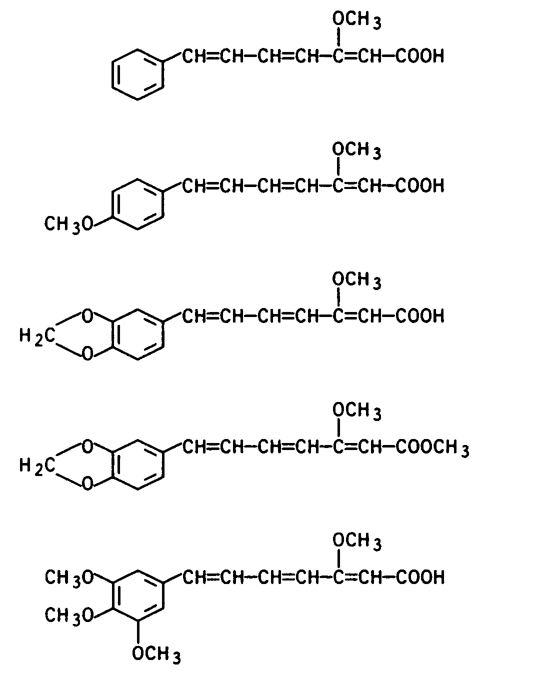

5. Kosmetische Zubereitungen, enthaltend 0,1 bis 10 Gew.-% der Verbindungen I gemäß Anspruch 1 oder 2 als Lichtschutzmittel.

## Patentansprüche (Patentansprüche für folgende(n) Vertragsstaat(en): ES)

1. Verfahren zur Herstellung von kosmetischen Zubereitungen, dadurch gekennzeichnet, daß man hierbei Arylpolyencarbonsäuren und ihre Derivate der allgemeinen Formel I 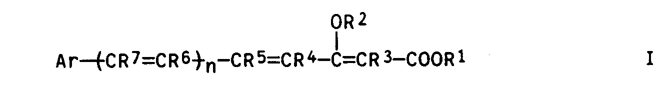 in der die Variablen folgende Bedeutung haben:
Ar ein Phenyl-, Biphenylyl- oder Naphthylrest, der durch ein bis drei C₁- bis C₄-Alkylgruppen, C₁- bis C₄-Alkoxygruppen, Hydroxylgruppen, Phenoxygruppen, Aminogruppen, welche durch C₁- bis C₄-Alkylgruppen mono- oder disubstituiert sein können, oder eine Methylendioxygruppe substituiert sein kann, wobei die Substituenten gleich oder verschieden sein können,
R¹ Wasserstoff, ein Alkalimetall- oder Ammoniumkation, eine C₁- bis C₂₀-Alkylgruppe oder eine C₂- bis C₂₀-Alkenylgruppe,
R² eine C₁- bis C₈-Alkylgruppe,
R³ bis R⁷ Wasserstoff oder C₁- bis C₄-Alkylgruppen und
n 0 oder 1
als Lichtschutzmittel verwendet.

2. Verfahren zur Herstellung von kosmetischen Zubereitungen nach Anspruch 1, dadurch gekennzeichnet, daß man hierbei Arylpolyencarbonsäuren und ihre Derivate I verwendet, bei denen die Variablen folgende Bedeutung haben:
Ar ein Phenylrest, der durch ein bis drei C₁- bis C₄-Alkylgruppen oder C₁- bis C₄-Alkoxygruppen oder eine Methylendioxygruppe oder eine Di(C₁- bis C₄-alkyl)aminogruppe substituiert sein kann,
R¹ Wasserstoff oder eine C₁- bis C₈-Alkylgruppe,
R² eine C₁- bis C₄-Alkylgruppe,
R³ bis R⁷ Wasserstoff oder eine Methylgruppe und
n 0 oder 1.

3. Verfahren zur Herstellung von kosmetischen Zubereitungen nach Anspruch 1 oder 2, dadurch gekennzeichnet, daß man 0,1 bis 10 Gew.% der Verbindungen I, bezogen auf die kosmetischen Zubereitungen, einsetzt.

## Claims (Claims for the following Contracting State(s): BE, CH, DE, FR, GB, IT, LI, NL)

1. The use of arylpolyenecarboxylic acids and their derivatives of the formula I 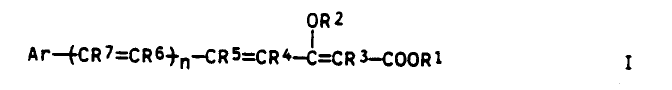 where
Ar is phenyl, biphenylyl or naphthyl, each of which can be substituted by one to three C₁-C₄-alkyl, C₁-C₄-alkoxy, hydroxyl or phenoxy groups, or amino groups which can be mono- or di-substituted by C₁-C₄-alkyl groups, or one methylenedioxy group, it being possible for the substituents to be identical or different,
R¹ is hydrogen, an alkali metal or ammonium cation, C₁-C₂₀-alkyl or C₂-C₂₀-alkenyl,
R² is C₁-C₈-alkyl,
R³ to R⁷ are each hydrogen or C₁-C₄-alkyl, and
n is 0 or 1
as sunscreen agents in cosmetic preparations.

2. The use of arylpolyenecarboxylic acids and their derivatives, I, where
Ar is phenyl which can be substituted by one to three C₁-C₄-alkyl or C₁-C₄-alkoxy groups or one methylenedioxy group or one di(C₁-C₄-alkyl)amino group,
R¹ is hydrogen or C₁-C₈-alkyl,
R² is C₁-C₄-alkyl,
R³ to R⁷ are each hydrogen or methyl, and
n is 0 or 1.

3. An arylpolyenecarboxylic acid or its derivative of the formula Ia 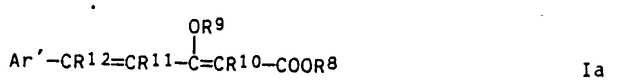 where Ar and Ar', R¹ and R⁸, R² and R⁹, and R³ to R⁵ and R¹⁰ to R¹² have the same meaning, with the exception of the following individual compounds for the arylpolyenecarboxylic acid and derivative Ia:

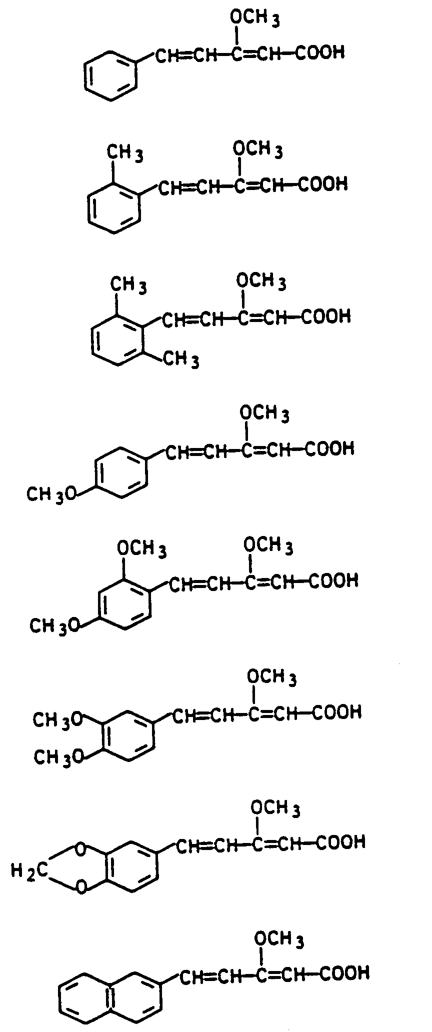

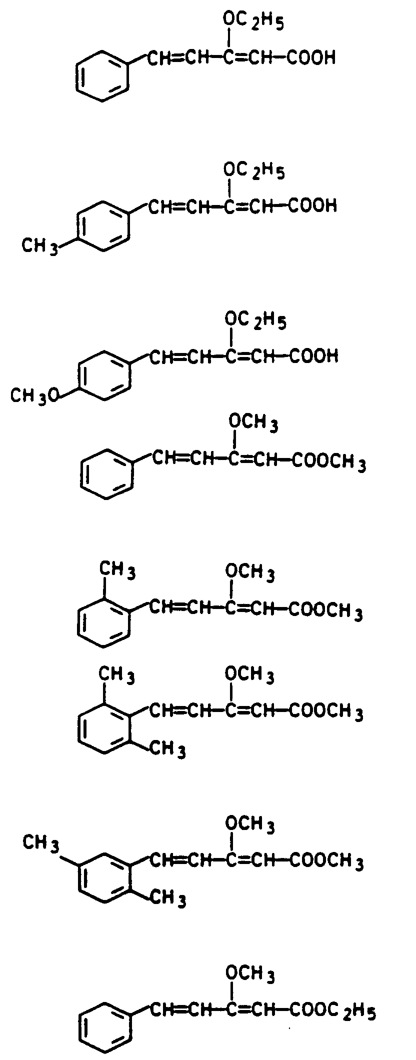

4. An arylpolyenecarboxylic acid or its derivative of the formula Ib 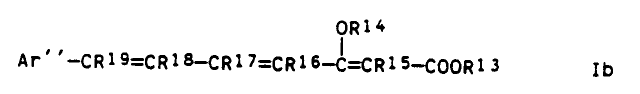 where Ar and Ar'', R¹ and R¹³, R² and R¹⁴, and R³ to R⁷ and R¹⁵ to R¹⁹ have the same meaning, with the exception of the following individual compounds for the arylpolyenecarboxylic acid and derivative Ib:

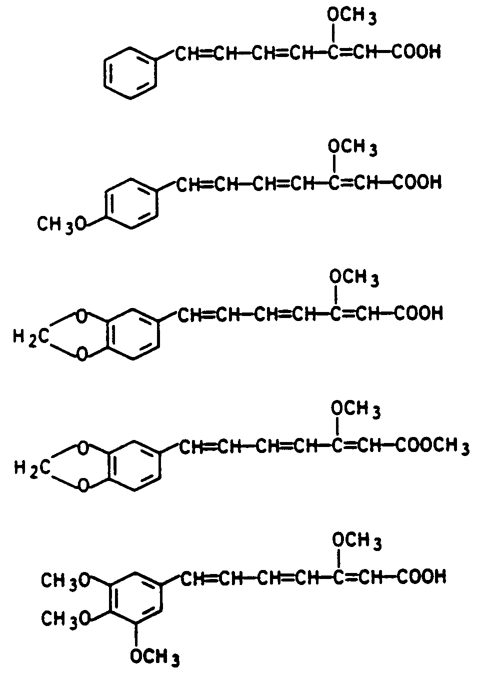

5. A cosmetic preparation containing from 0.1 to 10% by weight of a compound I as claimed in claim 1 or 2 as sunscreen agent.

## Claims (Claims for the following Contracting State(s): ES)

1. A process for producing cosmetic preparations, which comprises using arylpolyenecarboxylic acids and their derivatives of the formula I 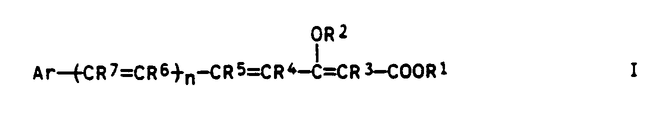 where
Ar is phenyl, biphenylyl or naphthyl, each of which can be substituted by one to three C₁-C₄-alkyl, C₁-C₄-alkoxy, hydroxyl or phenoxy groups, or amino groups which can be mono- or di-substituted by C₁-C₄-alkyl groups, or one methylenedioxy group, it being possible for the substituents to be identical or different,
R¹ is hydrogen, an alkali metal or ammonium cation, C₁-C₂₀-alkyl or C₂-C₂₀-alkenyl,
R² is C₁-C₈-alkyl,
R³ to R⁷ are each hydrogen or C₁-C₄-alkyl, and
n is 0 or 1
as sunscreen agents.

2. A process for producing cosmetic preparations as claimded in claim 1, which comprises using arylpolyenecarboxylic acids and their derivatives I, where
Ar is phenyl which can be substituted by one to three C₁-C₄-alkyl or C₁-C₄-alkoxy groups or one methylenedioxy group or one di(C₁-C₄-alkyl)amino group,
R¹ is hydrogen or C₁-C₈-alkyl,
R² is C₁-C₄-alkyl,
R³ to R⁷ are each hydrogen or methyl, and
n is 0 or 1.

3. A process for producing cosmetic preparations as claimed in claim 1 or 2, which comprises using from 0.1 to 10% by weight of the compounds I based on the cosmetic preparations.

## Revendications (Revendications pour l'(les) Etat(s) contractant(s) suivant(s): BE, CH, DE, FR, GB, IT, LI, NL)

1. Utilisation, comme agents protecteurs de la lumière dans des préparations cosmétiques, d'acides arylpolyéniques et leurs dérivés de formule générale 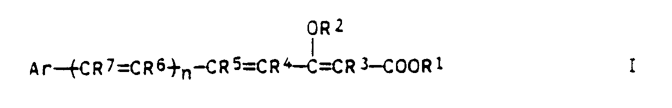 dans laquelle les variables ont les significations suivantes
Ar un reste phényle, biphényle ou naphtyle, qui peut être substitué par un à trois groupes alkyle en C1-C4, groupes alcoxy en C1-C4, groupes hydroxyle, groupes phénoxy, groupes amino, qui peuvent être mono ou disubstitués par des groupes alkyle en C1-C4, ou un groupe méthylènedioxy, les substituants pouvant être identiques ou différents,
R¹ hydrogène, un cation métal alcalin ou ammonium, un groupe alkyle en C1-C20 ou un groupe alcényle en C2-C20,
R² un groupe alkyle en C1-C8,
R³ à R⁷ hydrogène ou des groupes alkyle en C1-C4 et
n 0 ou 1.

2. Utilisation, comme agents protecteurs de la lumière dans des préparations cosmétiques, d'acides arylpolyéniques et leurs dérivés I selon la revendication 1, dans laquelle les variables ont les significations suivantes :
Ar un reste phényle qui peut être substitué par un à trois groupes alkyle en C1-C4 ou groupes alcoxy en C1-C4 ou un groupe méthylènedioxy ou un groupe di(alkyle en C1-C4)-amino,
R¹ hydrogène ou un groupe alkyle en C1-C8,
R² un groupe alkyle en C1-C4,
R³ à R⁷ hydrogène ou un groupe méthyle, et
n 0 ou 1.

3. Acides arylpolyéniques et leurs dérivés de formule générale Ia 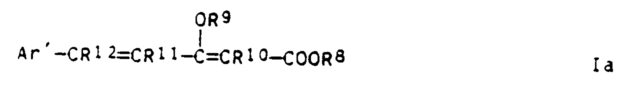 les variables Ar et Ar', R¹ et R⁸, R² et R⁹ ainsi que R³ à R⁵ et R¹⁰ à R¹² ayant la même signification, à l'exception des composés particuliers suivants pour les acides arylpolyéniques et dérivés Ia

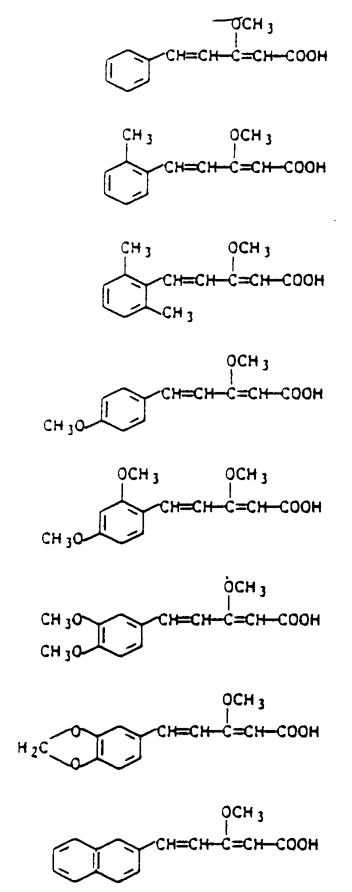

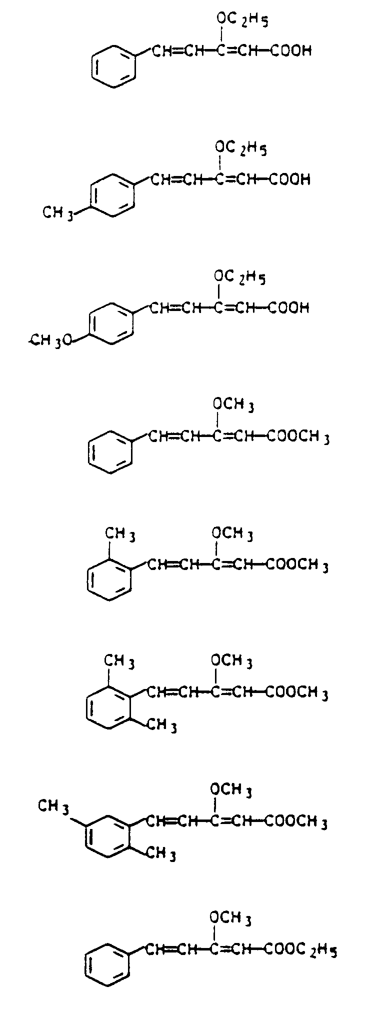

4. Acides arylpolyéniques et leurs dérivés de formule générale Ib 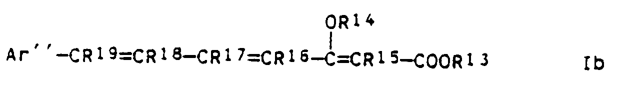 les variables Ar et Ar'', R¹ et R¹³, R² et R¹⁴ ainsi que R³ à R⁷ et R¹⁵ à R¹⁹ ayant la même signification, à l'exception des composés individuels suivants pour les acides arylpolyéniques et les dérivés Ib :

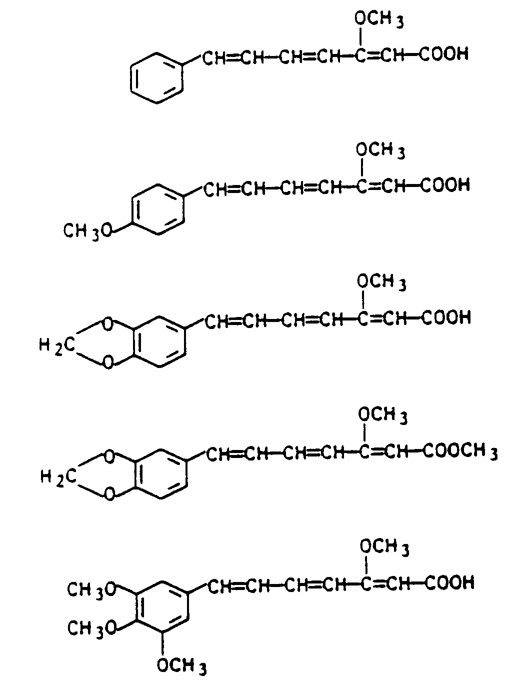

5. Préparations cosmétiques contenant 0,1 à 10 % en poids des composés I selon la revendication 1 ou 2, comme protecteurs de la lumière.

## Revendications (Revendications pour l'(les) Etat(s) contractant(s) suivant(s): ES)

1. Procédé de préparation de préparations cosmétiques, caractérisé par le fait que l'on utilise comme agent protecteur de la lumière, des acides arylpolyéniques et leurs dérivés de formule générale 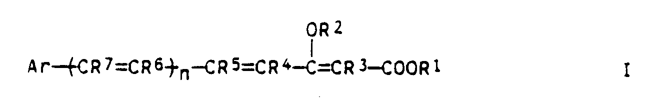 dans laquelle les variables ont les significations suivantes
Ar un reste phényle, biphényle ou naphtyle, qui peut être substitué par un à trois groupes alkyle en C1-C4, groupes alcoxy en C1-C4, groupes hydroxyle, groupes phénoxy, groupes amino, qui peuvent être mono ou disubstitués par des groupes alkyle en C1-C4, ou un groupe méthylènedioxy, les substituants pouvant être identiques ou différents,
R¹ hydrogène, un cation métal alcalin ou ammonium, un groupe alkyle en C1-C20 ou un groupe alcényle en C2-C20,
R² un groupe alkyle en C1-C8,
R³ à R⁷ hydrogène ou des groupes alkyle en C1-C4 et
n 0 ou 1.

2. Procédé de préparation de préparations cosmétiques selon la revendication 1, dans laquelle les variables ont les significations suivantes :
Ar un reste phényle qui peut être substitué par un à trois groupes alkyle en C1-C4 ou groupes alcoxy en C1-C4 ou un groupe méthylènedioxy ou un groupe di(alkyle en C1-C4)-amino,
R¹ hydrogène ou un groupe alkyle en C1-C8,
R² un groupe alkyle en C1-C4,
R³ à R⁷ hydrogène ou un groupe méthyle, et
n 0 ou 1.

3. Procédé de préparation de préparations cosmétiques selon la revendication 1 ou 2, caractérisé par le fait que l'on utilise 0,1 à 10 % en poids de composés I, rapportés à la préparation cosmétique.
